# EUROPEAN PATENT APPLICATION

(11) **EP 3 200 188 A1**
(43) Date of publication of application: **02.08.2017**
(21) Application number: 16382029.3
(22) Date of filing: 27.01.2016
(51) Int. Cl.: G10L 25/66, A61B 5/00

(54) **COMPUTER IMPLEMENTED METHODS FOR ASSESSING A DISEASE THROUGH VOICE ANALYSIS AND COMPUTER PROGRAMS THEREOF**

(71) Applicant: Telefonica Digital España, S.L.U., 28013 Madrid (ES)
(72) Inventor: Serrà Julià, Joan, 28013 Madrid (ES)
(74) Representative: Carlos Hernando, Borja

(57) **Abstract**

The methods comprises: a) computing from audio data uttered by at least one first mathematical audio transformation function to obtain a first time series representation of said audio data; b) computing at least one second mathematical audio transformation function, equal or different to the first mathematical function, over said obtained first time series representation to obtain a second time series representation of the audio data; c) segmenting said first and second time series representations into one or more timestamps; d) computing one or more different measures by using the one or more timestamps and the first and second time series representations; and e) using the one or more different computed measures to train a machine learning algorithm which computes a score for assessing said disease.

## Description

### Field of the invention

The present invention generally relates to methods for disease assessment. In particular, the invention relates to computer implemented methods, and computer programs, for assessing a disease, preferably Parkinson, through voice analysis.

### Background of the invention

Access to healthcare for Parkinson patients and other diseases that might be detectable via voice analysis, is mostly limited to people that have access to neurologists. Some patients might not have access to neurologists at all and some might have several barriers before they get adequate diagnosis and treatment. On average it takes five specialist (including non-neurologists) visits to be diagnosed with Parkinson and on average four years.

Document [1] discloses nonlinear speech analysis algorithms which reveal pathological characteristics in Parkinson's disease. Given a raw audio signal containing sustained vowel utterances, in the method proposed in this document dysphonia measure features (e.g., jitter, shimmer, harmonics to noise ratio), empirical mode decomposition excitation ratios, vocal fold excitation ratios, and classical spectral features like MFCCs and their derivatives are first extracted. Next, feature selection and regression are performed using least absolute shrinkage, selection operators, and classification and regression trees. Training is done with 42 Parkinson patients and a disease severity score is computed from the trained models.

Document [2] discloses an automatic evaluation of Parkinson's speech. Given a raw audio signal containing either spontaneous speech or specific speech tasks like reading words or syllable repetition, first groups of features (acoustic, prosodic, glottal, and openSmile features) are extracted. Acoustic features are based on a Gaussian mixture model of MFCCs and their first and second derivatives. Prosodic features are calculated over voiced and unvoiced fragments of audio, and include fundamental frequency energy, duration, and jitter. Glottal excitation features use a glottis model and linear predictive coding. OpenSmile features are a pool of features computed with the openSmile software package. To distinguish between diseased and healthy subjects, a support vector machine with linear kernel is trained on several functionals (statistical moments, minimum, maximum, percentiles, etc.) of the afore-mentioned features computed for 88 Parkinson patients and 88 controls. A three-stage prediction of the degree of severity of the patients is also done using the same feature and classification scheme. Individual accuracies per feature group and speech tasks are reported. A combination of speech tasks and feature groups is also studied but does not achieve the best accuracy.

Document [3] discloses a method for automatic evaluation of articulatory disorders in Parkinson's disease. The method, given a raw audio signal containing repeated diadochokinetic utterances, first segments the signal by locating high energy fragments, filtering the signal, and applying suitable thresholding, including Bayesian step change point detection. With this, the method is able to detect the timestamps of the initial burst of an utterance, vowel onsets, and occlusions. From this segmentation then a number of specific features are computed, including vowel similarity and variability quotients, onset to occlusion intervals, and speech timing measures. Such features are based on simple computations such as auto-correlation, time intervals, and low-level spectral characteristics. Features are summarized by statistical moments (mean and standard deviation) and a support vector machine classifier with radial basis function kernel is trained using 24 Parkinson patients (newly diagnosed/not already treated) and 22 controls.

Document [4] discloses a method using voiced/unvoiced transitions in speech as a potential bio-marker to detect Parkinson's disease. Said method, given a raw audio signal containing spontaneous speech, first locates transitions between sounds. To do so, a publicly available tool called 'Praat' that estimates the timestamps of such transitions using time-domain auto- and cross-correlation methods is used. Timestamps are then used to fragment the raw audio signal and get short excerpts from it (40 msec to the right and to the left of the timestamp). After that, standard MFCC and Bark band energy features are computed from those excerpts in frames of 20 msec lengths using a 10 msec hop. All features for the same audio file are then added to a separate array and summarized using statistical moments (mean, variance, skewness, and kurtosis). Finally, a support vector machine classifier with radial basis function kernel is trained with the obtained features for 50 Parkinson patients (under treatment) and 50 controls.

Some of the main problems of the above described solutions are:
- Scalability. They involve low number of users (below 100 patients with Parkinson in all cases) so they lack of objective data.
- Noisy and telephonic speech. There are insufficient strong studies considering these two sources.
- Free/unstructured speech. The vast majority of solutions work with specific voice utterances and exercises. This implies an active role of the patient/user, whereas a passive screening could be performed with free/unstructured/spontaneous speech.
- Parkinson's early stages. Few solutions exist for so-called "de novo" patients (recently diagnosed, not yet treated patients). No investigation with sensible populations has been done (subjects with LRKK2 genetic marker and/or with REM sleep behavior disorder).
- Active service. No solution is thought as a passive diagnosis/monitoring service. All previous solutions imply specific tasks/involvement of users.
- No patient-adaptive monitoring system available. Current solutions do not adapt to the specific, individual needs of the patient. They are not personalized.

References:
[1] A. Tsanas, et. al. 'Nonlinear speech analysis algorithms mapped to a standard metric achieve clinically useful quantification of average Parkinson's disease symptom severity', 2010, Journal of the Royal Society Interface, 8(59): 842-855.
[2] T. Bocklet, et. al. 'Automatic evaluation of Parkinson's speech - Acoustic, prosodic and voice related cues' 2013, Proc. of the Annual Conf. of the Int. Speech Communication Assoc. (INTERSPEECH), pp. 1149-1153.
[3] M. Novotny, et. al. 'Automatic evaluation of articulatory disorders in Parkinson's disease', 2014, IEEE/ACM Trans. on Audio, Speech, and Language Processing, 22(9): 1366-1378.
[4] J. R. Orozco-Arroyave, et. al. 'Voiced/unvoiced transitions in speech as a potential bio-marker to detect Parkinson's disease', 2015, Proc. of the Annual Conf. of the Int. Speech Communication Assoc. (INTERSPEECH), pp. 95-99.

### Description of the Invention

Embodiments of the present invention provide according to an aspect a computer implemented method for assessing a disease through voice analysis, wherein a computer system including one or more processors comprises the following steps:
a) computing from audio data uttered by a user a first mathematical audio transformation function to obtain a first time series representation of said audio data;
b) computing a second mathematical audio transformation function, equal or different to the first mathematical function, over said obtained first time series representation to obtain a second time series representation of the audio data;
c) segmenting said first and second time series representations into one or more timestamps;
d) computing one or more different measures by using the one or more timestamps and the first and second time series representations, said different measures including the computing of minimum and maximum values, percentiles, statistical moments, histograms, and/or entropies from the first and second time series representations and/or statistics about the one or more timestamps; and
e) using the one or more different computed measures to train a machine learning algorithm which computes a score for assessing said disease.

Embodiments of the present invention also provide according to another aspect a computer implemented method for assessing a disease through voice analysis, wherein a computer system including one or more processors comprises the following steps:
f) computing from audio data uttered by a user a plurality of different mathematical audio transformation functions to obtain a series of first time series representations of said audio data;
g) computing said plurality of different mathematical audio transformation functions over some or all of said obtained series of first time series representations to obtain a series of second time series representations of the audio data;
h) segmenting said series of first and second time series representations into one or more timestamps;
i) computing one or more different measures by using the one or more timestamps and the series of first and second time series representations, said different measures including the computing of minimum and maximum values, percentiles, statistical moments, histograms, and/or entropies from the time series representations, and/or statistics about the one or more timestamps; and
j) using the one or more different computed measures to train one or more machine learning algorithms which computes a score for assessing said disease.

Preferably, said disease to be assessed is Parkinson. However, the proposed methods are also suitable for other diseases that might be detectable via voice analysis.

According to an embodiment, the audio data is uttered by the user during a telephone conversation maintained by the user via a phone device. Alternatively, the audio data is uttered by the user in a controlled environment (room, sound-proof booth, etc.) via a microphone or a similar device.

According to an embodiment, previous to performing the above described steps a) or f), the audio data is recorded and stored in a memory or database, i.e. the proposed methods are executed off-line.

According to a preferred embodiment, the audio data further includes a label indicating whether the audio data comes from a diseased or a healthy user. In this case, the label may further indicate, if the label indicates that the user is a diseased user, an indication specifying the level of severity of the disease.

According to the invention, the mathematical audio transformation functions may include: a constant Q transform (CQT), Empirical mode decomposition (EMD), a Recurrence quantification analysis (RQA), and/or a fast Fourier transform (FFT).

According to an embodiment, the one or more different measures are computed over the entire first and second time series representations or series of first and second time series representations. Alternatively, according to another embodiment, the one or more different measures are computed over a specific part of the first and second time series representations or series of first and second time series representations. Even, according to yet another embodiment, the one or more different measures are computed over a specific timestamp.

The computed score may be communicated to the user either via a software application installed on a computer device or via a dedicated website.

Other embodiments of the invention that are disclosed herein include software programs to perform the method embodiment steps and operations summarized above and disclosed in detail below. More particularly, a computer program product is one embodiment that has a computer-readable medium including computer program instructions encoded thereon that when executed on at least one processor in a computer system causes the processor to perform the operations indicated herein as embodiments of the invention.

With the proposed invention, models are trained on an order of magnitude larger sample than the state of the art. Thus, the invention is more scalable than the state of the art, i.e., it theoretically provides better coverage to the full population. In addition model combinations with user and feature subsets provide a higher accuracy and stability of the results. They reduce the bias and variance of the invention.

Using and combining multiple feature representations allows for a better segmentation of the signal and more robust feature functionals.

Moreover, robust representations such as EMD and RQA allow working with noisy audios. Special attention is put to telephonic channel noise, with an extension specially dealing with that. No invention in the state-of-the-art deals with real telephonic speech.

Using techniques that are not in the state-of-the-art of Parkinson's detection through voice (such as CQT, EMD, or RQA) yields an improvement in accuracy. This is due both to the suitability of such techniques for analyzing nonlinear phenomena such as the one observed in voice pathologies, and also to the non-overlapping nature of the underlying aspects they cover, which makes their combination fruitful.

If sufficient data for a single user is available, models can be trained by taking that into account and comparing such user's disease progression with itself and other users. This way, a personalized, time-dependent disease progression score can be obtained.

Few works consider the case of free, spontaneous speech audio. The segmentation module of the proposed invention is a key module for dealing with such type of audio and allows the present invention to be applicable to the case of spontaneous speech.

No existing models consider the possibility of combining audio-derived features with information coming from demographics, medical records, or user self-assessment.

### Brief Description of the Drawings

The previous and other advantages and features will be more fully understood from the following detailed description of embodiments, with reference to the attached drawings, which must be considered in an illustrative and non-limiting manner, in which:
Fig. 1 is a schematic block diagram illustrating the different modules/units used by the present invention to assess a disease such as Parkinson.
Fig. 2 illustrates an example of two recurrence plots used in the RQA analysis. The top images refer to audio signals whereas the bottom images refer to recurrence plots. In addition left images refer to a noisy unvoiced frame whereas the right images refer to a periodic voiced frame.
Fig. 3 is a schematic block diagram illustrating the second layer of abstraction implemented by the proposed method to assess said Parkinson disease.
Figs. 4 and 5 are two block diagram describing two embodiments of the proposed method.

### Detailed Description of the Invention

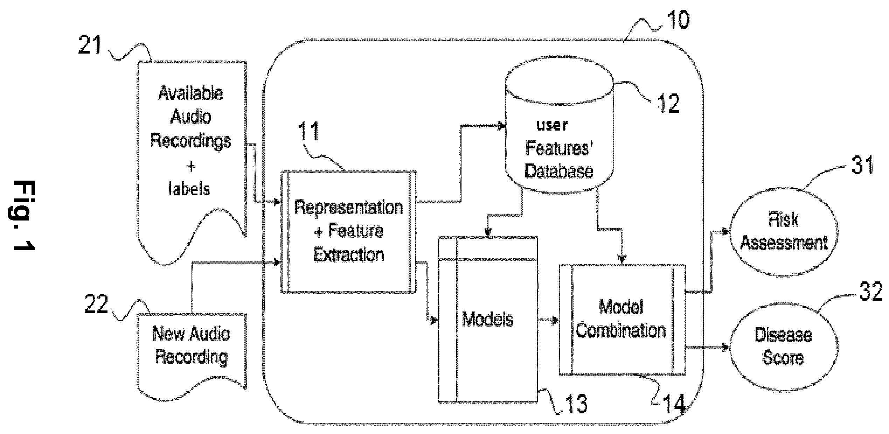

Fig. 1 illustrates a general block diagram of the proposed invention illustrating the different modules/units involved for assessing a disease such as Parkinson through voice analysis. According to the invention, the computer system 10, which includes one or more processors and at least one memory, implements an algorithm to perform two main tasks: train and prediction.

When training, raw audio data recordings previously uttered by a user (not illustrated) and their corresponding labels 21 are inputted to the computer system 10. According to the invention labels consist of a binary sign indicating whether the audio data comes from a diseased or a healthy user and, in case it is a diseased user, of a real number indicating the level of severity of the disease.

Alternatively, when predicting, only a single raw audio data recording 22 is input to the computer system 10. However, in this prediction case, instead of the audio data being previously recorded or stored in a memory or database of the computer system 10, or of another computer system (not illustrated either), the computer system 10 may directly receive the uttered audio data from the user, i.e. the computer system may perform the assessment in real time.

The output of the computer system 10 consists of a risk assessment score 31 and/or a disease progression score 32. The former gives an indication of the possibility of a user of having said disease (e.g. Parkinson). The latter gives an indication of the severity of the disease. Both outputs 31, 32 can be transmitted directly to a doctor or physician, or can be communicated directly to the user. Possibilities for the latter include a mobile APP, mobile web, an API, SDK, integration with telephone systems or a dedicated website.

According to a preferred embodiment, risk assessment score 31 consists of a real value between 0 and 1, indicating the probability of the user of having Parkinson based on the conducted analysis, whereas disease progression score 32 consists of an integer value between 0 and 32, equivalent to the Unified Parkinson's Disease Rating Scale (UPDRS) scale.

Alternatively, the output of the computer system 10 can also consist of intermediate speech analysis such as some feature evolution along time.

The audio data recording can be performed while the user maintains a phone conversation via a phone device (mobile, fixed, or VoIP) or in a controlled environment, for example a sound-proof booth.

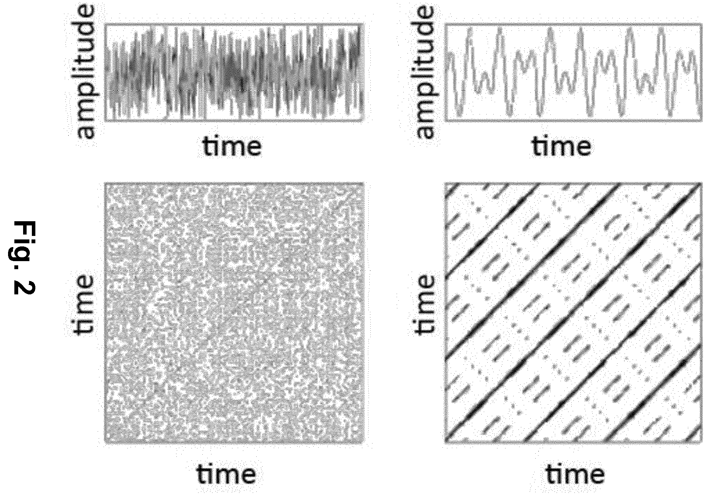

The computer system 10 includes a representation and feature extraction module 11 storing different mathematical audio transformation functions that can be used by the computer system 10 to compute at a first phase representation and features from the audio data signal(s) 21, 22 or from derived representations thereof. The following mathematical audio transformation functions can be used by the proposed invention:
- Constant Q transform (CQT): this representation gives information about the temporal evolution of the energy present in logarithmically-spaced frequency bands of the audio data signal(s).
- Empirical mode decomposition (EMD): this representation decomposes the raw audio data signal(s) into a number of intrinsic components, which unveil the underlying noises, periodicities, and trends of the audio data signal(s) 21, 22. All of these components either aggregated or individually, can serve as input to further stages of present invention.
- Recurrence quantification analysis (RQA): this representation essentially implies the analysis of recurrent similar states in a continuous embedding space derived from a time series. It typically implies the construction of a recurrence plot, which is used to assess such recurrent similar states, and the computation of a number of features derived from said recurrence plot. An example of two recurrence plots is shown in Fig. 2. RQA features, computed from the recurrence plot, include, but are not limited to, recurrence rate, determinism, laminarity, and trend.
- Classical audio processing features: these are based on the fast Fourier transform (FFT) of short-time frames of the audio signal. The features include, but are not limited to, Mel Frequency Cepstral Coefficients (MFCCs), Bark band energies, spectral moments and parameterizations, linear predictive coefficients, jitter, shimmer, and fundamental frequency energy. They also include their first and second frame-based derivatives. Initially, a time series (sequential) representation of them is considered.

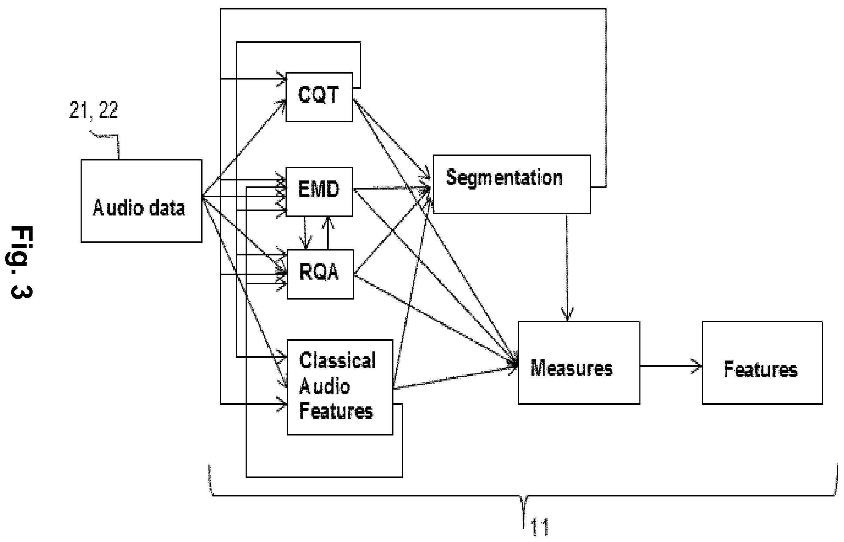

From the previous representations and features, the computer system 10 incorporates another phase of abstraction, consisting of applying again the mathematical audio transformation functions previously applied to a number of the already obtained representations and features (that is, a second computation of the mathematical audio transformation functions or functions, depending on the embodiment considered, is further implemented). Fig. 3 details an embodiment of this aspect. Essentially, the computer system 10 can take as input a given representation obtained in the first phase (e.g., the CQT) and compute features from it following another representation used in the first phase (e.g., RQA features). This deeper level of abstraction brings intuitively valuable features with which to train the proposed models that will be explained below.

Measures can be also applied on top of any given representation or feature (first or second phases), and be directly input to the proposed model schema. Measures may include minimum and maximum values, percentiles, statistical moments, histograms, entropies, and statistics about segment timestamps. Measures can be computed over an entire audio data recording 21, 22, over a segment, or over a specific part of the representation or feature. The last two are determined by a segmentation unit of the representation and feature extraction module 11.

The invention incorporates a segmentation stage working with representations and features, both at the individual and combined levels (i.e., performing segmentation with one feature at a time or performing segmentation with a combination of them). The segmentation unit is thought as an unsupervised, heuristics-based unit. It is based on thresholding of selected features/representations, especially RQA and EMD representations, but also on pattern-matching of transition events. The latter can be done on the basis of manually selected patterns or unsupervised inferred patterns. Patterns can be unsupervised inferred by considering time series motif finding algorithms or classical clustering algorithms adapted to the problem of repeated pattern discovery.

The computer system 10 includes also a user features' database 12 consisting of a series of feature arrays of high dimensionality, one per each audio data recording of each user being considered. These will be exploited by a models module 13 and also by the model combination module 14.

Models module 13 stores state-of-the-art machine learning algorithms, including but not limited to gradient boosted trees, random forests, support vector machines with linear and radial basis function kernels, deep neural networks, and recurrent neural networks.

Features are preferably standardized prior to being input to the models module 13. Models stored in the models module 13 are trained on subsets of the considered users and recordings. In addition, they can be trained on subsets of the considered features/representations. Subsets are chosen randomly. The same subset can be used for multiple models and the same machine learning algorithm can be used with different feature subsets.

The labels (ground truth data) used for training the models are: whether it is a healthy user or diseased user, and in case of being a diseased user, the UPDRS score. Thus, present invention considers two types of models: binary classifiers and real-valued regressors. For both cases present invention can derive the output values by model combination.

In the model combination module 14, the outputs of all considered models are combined in order to derive the final output. For both risk assessment 31 and disease progression scores 32 present invention follows a voting strategy, where each model's output contributes with a pre-trained weight to the final decision. It has to be noted that this implies that, for binary classifiers, after adding up their weighted outputs, a real-valued single number is obtained. This corresponds to the risk assessment score 31, yielded in terms of a probability. The combined value for the regressors already lead to a single real-valued number in the range of the UPDRS score present invention tries to predict.

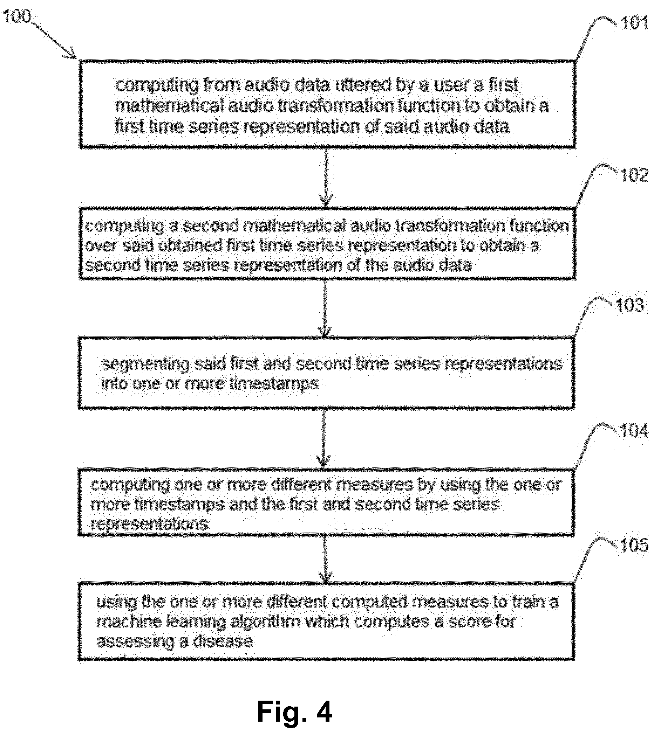

With reference now to Fig. 4, therein it is illustrated an embodiment of the proposed method for assessing a disease that might be detectable via voice analysis such as Parkinson. The method 100 starts by computing, step 101, from audio data uttered by a user a first mathematical audio transformation function to obtain a first time series representation of said audio data. Then, step 102, a second mathematical audio transformation function, equal or different to the first mathematical function, is computed over said obtained first time series representation to obtain a second time series representation of the audio data. At that time, step 103, said first and second time series representations are segmented into one or more timestamps and then, step 104, one or more different measures (e.g. maximum values, percentiles, statistical moments, histograms, and/or entropies from the time series representations, and/or statistics about the one or more timestamps) are computed by using the one or more timestamps and the first and second time series representations. Finally, step 105, the one or more different computed measures are used to train a machine learning algorithm which computes a score (either a risk score or a disease progression score) for assessing said Parkinson disease.

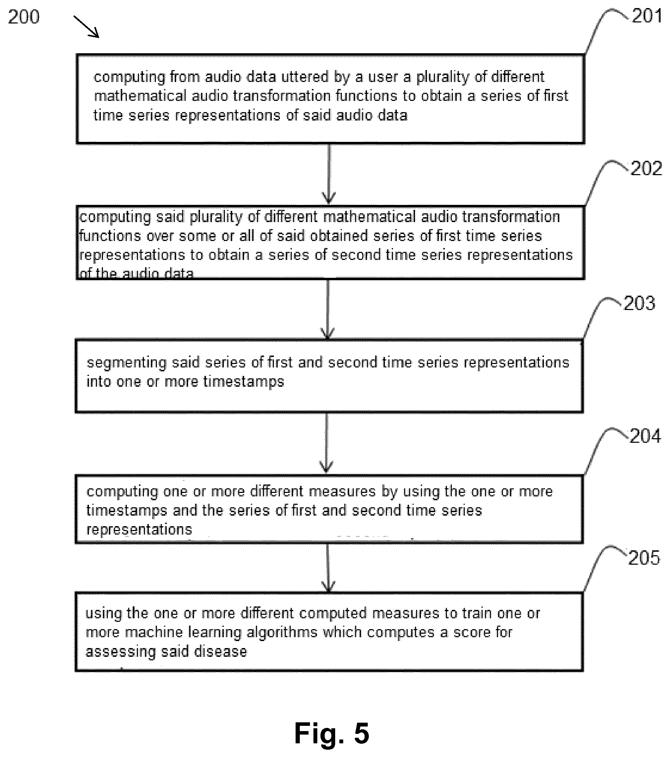

Fig. 5 illustrates another embodiment of the proposed method for assessing Parkinson. In this case, instead of computing a single mathematical audio transformation function, a plurality of different mathematical audio transformation functions are computed, step 201, from the audio data uttered by the user to obtain a series of first time series representations of said audio data. Then, step 202, a plurality of different mathematical audio transformation functions are also computed over some or all of said obtained series of first time series representations to obtain a series of second time series representations of the audio data. Once the series of first and second time series representations are computed this are segmented, step 203, into one or more timestamps, and one or more different measures computed thereof, step 204. Finally, the computed measures are used to train one or more machine learning algorithms which compute the final score for assessing the disease.

Following a detailed embodiment of the training phase implemented by the proposed method will be detailed:
a. From raw audio data recordings and associated labels 21 the CQT is computed obtaining a multidimensional time series representation R1.
b. From the raw audio data recordings and associated labels 21 the EMD is computed obtaining a multidimensional time series representation R2.
c. From the raw audio data recordings and associated labels 21 RQA features are computed in a sliding window obtaining a multidimensional time series representation R3.
d. From the raw audio data recordings and associated labels 21 Classical Audio Processing features are computed in a sliding window obtaining a multidimensional time series representation R4.
e. From R1 Classical Audio Processing features are computed in a sliding window, bypassing the typical FFT module, obtaining a multidimensional time series R5.
f. From R1 RQA features are computed in a sliding window obtaining a multidimensional time series representation R6.
g. From R1 the EMD is computed obtaining a multidimensional time series representation R7.
h. From R2 RQA features are computed in a sliding window obtaining a multidimensional time series R8.
i. From R3 the EMD is computed obtaining a multidimensional time series representation R9.
j. Representations R1 to R9 are inputted to the segmentation module which partitions the time series into coherent, homogeneous blocks and yields one or more series of segmentation timestamps TX.
k. From said timestamps TX some of the outlined representations R1-R9 are re-computed. Present invention can focus on the content at the transition itself or either on the content between transitions. Refined representations R1-R9 are obtained.
l. From timestamps TX and R1-R9 representations different measures F1-F9 are computed. These measures are quantities summarizing representations R1-R9 either at the transitions delimited by timestamps TX or between these. For each time series representation R1-R9 a vector of real numbers F1-F9 is obtained.
m. Vectors F1-F9 are concatenated into a single vector FU. One vector per audio file is obtained.
n. All single vectors FU from all audio data files are combined, yielding a feature matrix FM.
o. Feature matrix FM, together with the labels, is used to train a series of machine learning models MX. Only sections, i.e., randomly selected rows and columns, of feature matrix FM are used for a single model. The same or different machine learning algorithms can operate on the same or different sections of feature matrix FM. Identifiers for selected columns are stored together with the machine learning models MX and constitute a preprocessing step of it.
p. The full feature matrix FM, together with the machine learning models MX and labels, is used to train the weights of the output of each machine learning models WM. To do so, a separate validation set can be used.

With reference to the prediction phase, a detailed embodiment would be as follows:
a. From a single spontaneous speech audio data (either previously recorded or analysed in real time) the same previously detailed steps a) to m) are implemented.
b. Then, the single vector FU is inputted to every machine learning model MX and a prediction output score PM is calculated.
c. Outputs PM are weighted according to the machine learning models WM and summed up to obtain the risk assessment score 31 or the disease score 32, depending on the labels used to train the machine learning models MX and WM.

According to the invention, the audio data used can be single or multiple per user. These can include spontaneous, free speech, or targeted specific speech tasks, including but not limited to sustained vowel phonations, word and sentence reading, and diadochokinetic utterances.

Also, users that form the training basis come from multiple user groups, including both healthy users and patients with Parkinson. The latter can be subdivided into users with LRKK2 genetic marker, users with sleep disorder, diagnosed but untreated users, and diagnosed and treated users with different Parkinson's disease progression stages.

Initial input labels could be expanded to demographics data facilitated by the patient, medical assessment variables (including but not limited to type of medication, dose, year of diagnosis, patient's age), and self-reported variables (including but not limited to emotional textual descriptions, illness-specific subjective evaluations, and self-administered questionnaires).

Input audio data can go beyond clean audio recordings. In particular, present invention can consider dynamically-compressed audio, perceptually-compressed audio (e.g., MP3), distorted audio, telephonic channel audio, environmentally-noisy audio, incomplete audio (e.g., with silences or extreme noises in short-time chunks of the signal), etc.

Furthermore, the input audio data may be preprocessed to enhance its quality and remove noise.

Deployed features or representations can be component-wise and instance-wise normalized both at intermediate or final stages.

Levels of assessment of the Parkinson's disease include but are not limited to UPDRS and Hoehn & Yahr scores, and intelligibility indices.

The risk assessment score 31 can be based on different concepts, including but not limited to probabilities, medical scores, and binary values. The disease progression score 32 can be based on different concepts and strategies, including but not limited to UPDRS, Hoehn & Yahr scores, and hospital-specific scales. At the same time, it can be based on the user itself, in case this has provided previous data to the computer system 10, or other users, be these in a similar or a very different condition as compared to the user under test.

In addition, more feedback loops can be added in the feature/representation extraction process. For instance RQA with RQA itself or Classical Audio Processing features from RQA-derived representations. Feedback loops can operate over all possible dimensions of the representation or a subset of these.

The models considered by present invention can be expanded also to logistic regression, nearest neighbors models, and Bayesian classifiers, among others. In addition, models can include specifically-designed capabilities to identify distortionless features, or those less prone to be affected by distortion, or identifying which features to ignore under certain suboptimal conditions of the audio recording or the telephonic channel.

Feature subset selection prior to model training can also be done manually or based on some existing feature selection criteria, including but not limited to Chi square, Gini coefficient, and recursive feature selection.

In the case a sample for a specific user group or audio task may be considered too small for training the proposed models, the synthetic minority oversample technique can be considered.

Besides, the segmentation module can be enhanced by considering a supervised approach. In addition, a phoneme recognition engine can also be employed in order to derive a phoneme-based segmentation of free speech and the posterior probability of the inferred phonemes.

Apart from weighted voting, model combination can be done with known techniques like stacking, boosting, or bagging.

While the foregoing is directed to embodiments of the present invention, other and further embodiments of the invention may be devised without departing from the basic scope thereof. For example, other aspects may be implemented in hardware or software or in a combination of hardware and software.

Additionally, the software programs included as part of the invention may be embodied in a computer program product that includes a computer useable medium. For example, such a computer usable medium can include a readable memory device, such as a hard drive device, a flash memory device, a CD-ROM, a DVD/ROM, or a computer diskette, having computer readable program code segments stored thereon. The computer readable medium can also include a communications link, either optical, wired, or wireless, having program code segments carried thereon as digital or analog signals.

The scope of the present invention is determined by the claims that follow.

## Claims

1. A computer implemented method for assessing a disease through voice analysis, wherein a computer system including one or more processors comprises the following steps:
a) computing from audio data uttered by a user a first mathematical audio transformation function to obtain a first time series representation of said audio data;
b) computing a second mathematical audio transformation function, equal or different to the first mathematical function, over said obtained first time series representation to obtain a second time series representation of the audio data;
c) segmenting said first and second time series representations into one or more timestamps;
d) computing one or more different measures by using the one or more timestamps and the first and second time series representations, said different measures including the computing of minimum and maximum values, percentiles, statistical moments, histograms, and/or entropies from the first and second time series representations and/or statistics about the one or more timestamps; and
e) using the one or more different computed measures to train a machine learning algorithm which computes a score for assessing said disease.

2. A computer implemented method for assessing a disease through voice analysis, wherein a computer system including one or more processors comprises the following steps:
f) computing from audio data uttered by a user a plurality of different mathematical audio transformation functions to obtain a series of first time series representations of said audio data;
g) computing said plurality of different mathematical audio transformation functions over some or all of said obtained series of first time series representations to obtain a series of second time series representations of the audio data;
h) segmenting said series of first and second time series representations into one or more timestamps;
i) computing one or more different measures by using the one or more timestamps and the series of first and second time series representations, said different measures including the computing of minimum and maximum values, percentiles, statistical moments, histograms, and/or entropies from the time series representations, and/or statistics about the one or more timestamps; and
j) using the one or more different computed measures to train one or more machine learning algorithms which computes a score for assessing said disease.

3. The computer implemented method of any of previous claims, wherein said disease is Parkinson.

4. The computer implemented method of any of previous claims, wherein the audio data being uttered during a telephone conversation maintained by the user via a phone device or being uttered in a controlled environment via a microphone.

5. The computer implemented method of claim 4, wherein the audio data being recorded and stored in a memory or database previous to the execution of step a) or step f).

6. The computer implemented method of any of previous claims, wherein the audio data further includes a label indicating whether the audio data comes from a diseased or a healthy user.

7. The computer implemented method of claim 6, further comprising in case said label indicating that the user being a diseased user an indication specifying the level of severity of the disease.

8. The computer implemented method of claim 6 or 7, wherein the computed score is a disease progression score 32.

9. The computer implemented method of claim 1, wherein the computed score is a risk assessment score 31.

10. The computer implemented method of claim 1 or 2, wherein the mathematical audio transformation functions including a constant Q transform, or CQT, an Empirical mode decomposition, or EMD, a Recurrence quantification analysis, or RQA, and/or a fast Fourier transform, or FFT.

11. The computer implemented method of claim 1 or 2, wherein the one or more different measures being computed over the entire first and second time series representations or series of first and second time series representations, over a specific part thereof, or over a specific timestamp.

12. The computer implemented method of claim 1 or 2, further comprising communicating the computed score to the user via a software application installed on a computer device or via a dedicated website.

13. A computer program product comprising software program code instructions which when loaded into a computer system controls the computer system to perform each of the methods steps of claims 1 to 12.
